# EUROPEAN PATENT APPLICATION

(11) **EP 4 186 497 A1**
(43) Date of publication of application: **31.05.2023**
(21) Application number: 21210877.3
(22) Date of filing: 26.11.2021
(51) Int. Cl.: A61K 31/12, A61K 31/353, A61K 31/385, A61P 19/02, A61P 31/14

(54) **ALPHA LIPOIC ACID, EPIGALLOCATECHIN 3-GALLATE, AND CURCUMIN AS A MEDICAMENT FOR THE TREATMENT OF ARTHRITIS OR SARS-COV-2 INFECTION**

(71) Applicant: Bevec, Dorian, 61231 Bad Nauheim (DE); BIOTANK Co. Ltd., Dongjak-gu, Seoul (KR)
(72) Inventor: Bevec, Dorian, 61231 Bad Nauheim (DE); Falarzik, Andreas, 44867 Bochum (DE); Jäger, Frank, 44793 Bochum (DE); Freiherr von Ziegesar, Ralf, 80802 München (DE)
(74) Representative: Steiniger, Carmen

(57) **Abstract**

The invention relates to the use of the solubilized Alpha Lipoic Acid, solubilized Epigallocatechin 3-gallate, and solubilized Curcumin in combination as (a) therapeutic agent(s) for the prophylaxis and/or treatment of arthritis including, without limitation, fibrotic disease, inflammatory disease, neurodegenerative disease, autoimmune disease, or heart and vascular disease as a consequence of arthritic pathophysiology, and viral infections including SARS-CoV-2 infections.

The invention further relates to the use of the mentioned solubilized therapeutic agent(s) for for oral administration, and for inhalative administration.

## Description

The invention relates to the use of Alpha Lipoic Acid solublisate, Epigallocatechin 3-gallate solubilisate, and Curcumin solubilisate in combination as therapeutic medicament for the prophylaxis and/or treatment of any type of Arthritis.

The invention further relates to the use of Alpha Lipoic Acid solubilisate, Epigallocatechin 3-gallate solubilisate, and Curcumin solubilisate in combination as therapeutic medicament for the prophylaxis and/or treatment of viral infections, particulary of Corona-virus infections and subsequent lung diseases.

The invention further relates to the use of the therapeutic combination for oral or inhaled administration.

Autoimmune disease refers to any of a group of diseases or disorders in which tissue injury is associated with a humoral and/or cell-mediated immune response to body constituents or, in a broader sense, an immune response to self. The pathological immune response may be systemic or organ specific. That is, for example, the immune response directed to self may affect joints, skin, myelin sheath that protects neurons, kidney, liver, pancreas, thyroid, adrenals, and ovaries.

It has long been known that immune complex formation plays a role in the etiology and progression of autoimmune disease. For example, inflammation in patients with Arthritis has long been considered to involve phagocytosis by leukocytes of complexes of antigen, antibody and complement-immune complexes. However, only now it is being recognized that inflammation caused by immune complexes in the joints (Arthritis), the kidneys (glomerulonephritis), and blood vessels (vasculitis) is a major cause of morbidity in autoimmune diseases. Increased immune complex formation correlates with the presence of antibodies directed to self or so-called autoantibodies, and the presence of the latter can also contribute to tissue inflammation either as part of an immune complex or unbound to antigen (free antibody).

Another aspect of the etiology and progression of autoimmune disease is the role of proinflammatory cytokines. Under normal circumstances, proinflammatory cytokines such as tumor necrosis factor (TNF) and interleukin-1 (IL-1) play a protective role in the response to infection and cellular stress. However, the pathological consequences which result from chronic and/or excessive production of TNF and IL-1 are believed to underlie the progression of many autoimmune diseases such as rheumatoid Arthritis, Crohn's disease, inflammatory bowel disease, and psoriasis.

Inflammation is the final common pathway of various insults, such as infection, trauma, and allergies to the human body. It is characterized by activation of the immune system with recruitment of inflammatory cells, production of pro-inflammatory cells and production of pro-inflammatory cytokines. Most inflammatory diseases and disorders are characterized by abnormal accumulation of inflammatory cells including monocytes/macrophages, granulocytes, plasma cells, lymphocytes and platelets. Along with tissue endothelial cells and fibroblasts, these inflammatory cells release a complex array of lipids, growth factors, cytokines and destructive enzymes that cause local tissue damage.

TNF has several biologic activities that are important in homeostasis as well as in pathophysiological conditions. The main sources of TNF are monocytes-macrophages, T-lymphocytes and mast cells. The finding that anti-TNF antibodies are effective in the treatment of patients suffering from rheumatoid arthritis intensified the interest to find new TNF inhibitors as possible potent medicaments for arthritis. Rheumatoid arthritis is an autoimmune chronic inflammatory disease characterized by irreversible pathological changes of the joints. In addition to rheumatoid arthritis, TNF antagonists are also applicable to several other pathological conditions and diseases such as spondylitis, osteoarthritis, gout and other arthritic conditions.

Symptoms and signs of inflammation associated with specific conditions include:
- rheumatoid arthritis:- pain, swelling, warmth and tenderness of the involved joints; generalized and morning stiffness;
- scleroderma:- Raynaud's disease; swelling of the hands, arms, legs and face; skin thickening; pain, swelling and stiffness of the fingers and knees, gastrointestinal dysfunction, restrictive lung disease; pericarditis; renal failure;
- other arthritic conditions having an inflammatory component such as rheumatoid spondylitis, osteoarthritis, septic arthritis and polyarthritis:- fever, pain, swelling, tenderness.

There are more than 100 types of Arthritis known, comprising e.g. rheumatoid arthritis, osteoarthritis, fibromyalgia, gout, systemic Lupus Erythematodus.

The causative mechanism of many diseases is the over activity of a biological pathway. A medication that can reduce the activity of the biological pathway can be effective in the prophylaxis and/or treatment of the disease caused by the over activity of the biological pathway. Similarly, the causative mechanism of many diseases is the over production of a biological molecule. A medication that can reduce the production of the biological molecule or block the activity of the over produced biological molecule can be effective in the prophylaxis and/or treatment of the disease caused by the over production of the biological molecule.

Conversely, the causative mechanism of many diseases is the under activity of a biological pathway. A medication that can increase the activity of the biological pathway can be effective in the prophylaxis and/or treatment of the disease caused by the under activity of the biological pathway. Also, similarly the causative mechanism of many diseases is the under production of a biological molecule. A medication that can increase the production of the biological molecule or mimic the biological activity of the under produced biological molecule can be effective in the prophylaxis and/or treatment of the disease caused by the under production of the biological molecule.

The object of the present invention is solved by the teaching of the independent claims. Further advantageous features, embodiments and details of the invention are evident from the dependent claims, the description, and the examples of the present application.

**Alpha** Lipoic Acid (ALA, also known as (R)-5-(1,2-Dithiolan-3-yl)pentanonic acid 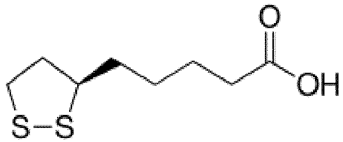 as an ingredient has been compounded for more than 25 years as an injection, suppository, topical and troche formulation. ALA is compounded for intravenous administration to treat diabetes and diabetic nephropathy. Available clinical reports revealed no serious safety concerns. However ALA is unstable in aqueous formulations. We have developed a solubilisated formulation of ALA that can also be used via oral administration.

ALA is an endogenous human metabolite, essential for orchestrating many metabolic pathways in humans. It has antioxidant and anti-inflammatory properties and improves muscle strength and regeneration following strenuous exercise.

ALA is believed to prevent skeletal muscle degradation and aid recovery, this effect being potentially due to reduced inflammation and oxidative stress. ALA is a very well researched compound, with more than 5 000 publications in scientific professional journals. Therefore ALA is considered to be a safe and potentially beneficial ingredient.

### Popular use

ALA is used by athletes as a nutritional supplement to improve strength, muscle growth, endurance and recovery.

### Medical use

ALA is prescribed to treat peripheral neuropathies in diabetic patients. Its efficacy and safety is well established.

### Scientific evidence

ALA reduces oxidative stress directly due to its scavenging abilities and its metal-chelating activity. It acts indirectly by increasing the expression of antioxidant enzymes like superoxide dismutase (SOD), heme oxygenase-1 and by regenerating glutathione, vitamins C and E.

### The polyphenol Epigallocatechin 3-gallate (EGCG) is the major catechin present in green tea.

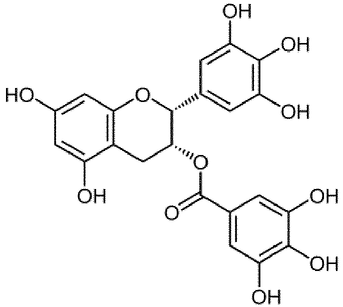

### Neurology / Anti-aging

EGCG reduces neuroinflammation, anxiety, activates working memory and improves attention. EGCG slows Alzheimer's progression due to its inhibition of acetylcholinesterase and butyryl-cholinesterase and showed promise in Parkinson's disease. EGCG reduces stroke risk in male non-smokers. EGCG reduces brain inflammation and neuronal damage in an autoimmune encephalomyelitis model.

### Antioxidant

EGCG has potent antioxidant activity. EGCG is able to scavenge ROS, which activate NF-κB to upregulate expression of inflammatory cytokines and inflammation-related enzymes. EGCG protects vascular endothelial cells from oxidative stress-induced damage by targeting the autophagy-dependent PI3K-AKT-mTOR pathway.

### Anti-inflammatory

EGCG affects the expression of inflammation-associated genes like TNF, IL-1β, MMPs and COX-2. EGCG decreases inflammation by affecting the TLR4 signaling pathway. EGCG suppresses the phosphorylation of IKKβ, and the activation of TRAF6 and NF-κB. EGCG treatment inhibits IL-1β, IL-6, TNF, and monocyte chemotactic protein-1 mRNA expression and suppresses gene expression of TGF-β1 and MMP-9.

### Metabolic disorders

Green tea lowers fasting blood glucose concentration and may lower risk of developing diabetes. This effect is associated with EGCG which reduces hepatic glucose production. EGCG inhibits α-glucosidase and has its hypoglycemic effect via the PI3K/AKT signaling pathway. EGCG increases concentrations of the circulating anti-inflammatory cytokine, interleukin-10, and delays type 1 diabetes onset.

### Curcumin (also known as diferuloylmethane) and Tetrahydrocurcumin

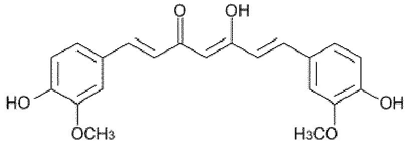

Curcumin is a compound extracted from the plant turmeric.

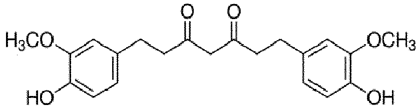

Tetrahydrocurcumin (1,7-Bis-(4-hydroxy-3-methoxyphenyl)-3,5-heptandion is a compound extracted from the plant turmeric.

Curcuminoids are used as a traditional herbal medicinal products, herbal supplement, food flavoring and food coloring. Curcuminoids have been shown to be anti-inflammatory, and antioxidant.

Curcumin protects cells from reactive oxygen species (ROS) by scavenging ROS and nitric oxide - induced free radicals.

Curcumin induces expression of cytoprotective proteins or antioxidant enzymes such as superoxide dismutase, catalase, glutathione reductase, glutathione peroxidase, heme oxygenase 1, and glutathione-S-transferase.

Curcumin reduces exaggerated inflammation by interacting with pro-inflammatory interleukin production and decreasing the action of phospholipase A2, cyclooxygenase-2, and 5-lipoxygenase. Its anti-inflammatory action is due to its modulation of NFκB, Nrf2, iNOS, IL-1α, IL-6, TNF, IL-1β.

Curcumin is able to cross the blood-brain barrier and reduces oxidative stress in the brain. Curcumin is able to increase the intracellular glutathione (GSH) levels - increased intracellular GSH levels in Alzheimer's patients seem to be meaningful since a low cellular GSH level is instrumental in Alzheimer's disease pathogenesis.

Curcumin decreases exaggerated TNF, MCP-1, glucose and glycosylated haemoglobin demonstrating immunomodulatory effects in obesity and insulin resistance. Curcumin reduces glucose-6-phosphatase and phosphoenolpyruvate carboxykinase activities lowering glucose levels in blood and increasing glucose tolerance. Curcumin also reduces plasma free fatty acid, cholesterol, and triglyceride levels and increases hepatic glycogen and skeletal muscle lipoprotein lipase (LPL). Curcumin has been shown to prevent skeletal muscle degradation, this effect being potentially due to reduced oxidative stress and the inhibition of inflammation and fibrosis.

Curcumin is a very well researched compound, with more than 13 600 publications in scientific professional journals. Therefore curcumin is considered to be a safe and potentially beneficial ingredient.

### Traditional use in Europe

The turmeric root is approved by the European Medicines Agency for the relief of digestive disturbances, such as feelings of fullness, slow digestion and flatulence.

All three products have in common a very low bioavailability, which precluded further use in human diseases. We have developed a novel formulation which solves this problem and dramatically increases the cellular availability for ALA, EGCG, and Curcumin.

A broad variety of substances are known for which potentially beneficial effects on human health have been found in experimental settings. The use of many of them, however, has been seriously limited by the poor cell- or bioavailability that can be achieved by application forms known in the state-of-the-art. In pharmacology, cell- or bioavailability is a parameter that indicates the fraction of an administered dose of unchanged drug that finally becomes available in the systemic circulation for the desired pharmacological effects. Poor cell- or bioavailability is often due to a poor water solubility, respectively the lipophilic nature of the active agent to be administered. Hence, the use of such substances as a dietary supplement or as a pharmaceutically active agent is impaired when using standard dosage forms.

There is a variety of approaches for improving the solubility of such agents and in many cases also their cell- or bioavailability by using solubilization techniques. Herein the solubility of an agent in a medium is augmented by adding an additional third substance. These third substances are referred to as solubilizers (solubilizing agents), substances that may for example build a complex with the substance to be solubilized. Examples for such chelating agents are sodium benzoate and sodium salicylate. Another mechanism of action of solubilizers is the augmentation of the dissolving capacity of the solvent, for example by disturbing the cluster structure of water. Examples for such structure breakers are glycerol (glycerin) and macrogols (polyethylene glycol, PEG).

A third solubilization mechanism are micelle and liposome application technologies. Herein the substance to be delivered is enclosed in a spherical aggregate of surfactant molecules. These molecules are characterized by a polar head group and a long nonpolar chain. When given into an aqueous medium these molecules tend to associate by aggregating to spherical structures by orienting the polar head group towards the surrounding medium and the nonpolar chain towards the interior of the spheres. When these spheres consist of only one layer of such amphiphilic molecules they are referred to as micelles. Depending on the nature of the amphiphilic molecule and the reaction conditions it is also possible to form spheres with more than one layer. Herein a second layer is formed inside the outer layer of the sphere, the nonpolar groups of this second layer being oriented towards the nonpolar groups of the outer layer, and the polar head groups being oriented towards the interior of the sphere. Such aggregates are referred to as liposomes. In their structure they resemble the lipid bilayer of the cell membrane. There are also multi-layered liposomes in which at least two liposomal spheres are formed concentrically around one another, thus building a multispherical aggregate. When given in a lipophilic medium these substances tend to build inversed spherical structures where the lipophilic chain is oriented towards the solution medium and the other layers are arranged accordingly.

Different uses of such loaded spheres have been described in the art, among them the usage as a dosage form for the application of lipophilic substances and/or for increasing the cell- or bioavailability of the enclosed substance. In micelles, the enclosed nonpolar substance concentrates in the interior space of the sphere toward which the nonpolar chains of the amphiphilic molecules are oriented. In liposomes, however, the interior space of the spheres is an aqueous, respectively hydrophilic medium. It can serve for packaging hydrophilic molecules. Poorly water-soluble, respectively lipophilic molecules, however, gather mostly in between the lipophilic structures of the liposomal layers.

A micelle-based solubilisate for solubilizing dietary supplements is known for ubiquinone Q10 (WO03/007907A1) or curcumin (WO2014/094921 A1). Therein an emulsifier with a HLB (hydrophilic-lipophilic balance) value of 9-16 or 13-18 is used, respectively. Polysorbate (Tween) 20 or 80 is preferred. WO2007/103435A1 discloses that an increased bioavailability of curcuminoids (curcumin ester derivatives) can be achieved by admixing micelles, microemulsions or microencapsulated oils as well as an antioxidant and advisably a glucuronidation inhibitor to the curcuminoids. The use of a surfactant such as poloxamers or polysorbate 20, polysorbate 60, polysorbate 80 is particularly preferred. Glucuronidation inhibitors, however, inhibit also the proper metabolization and consequently the elimination of other drugs or endogenous substances. From empiric pharmacokinetic measurements it is known that the organism can absorb micelles as well as liposomes in the gastrointestinal tract via the intestinal villi. However, their degree of absorption seems to be rather variable and therefore these methods have met a mixed success for augmenting the bioavailability of the enclosed compound. The transport, respectively the absorption rate over the cell membrane is an intrinsic characteristic for each substance, depending on a variety of factors such as molecule size, degree of lipophilicity and the presence of suitable transporter molecules inside the cell membrane. For many compounds these parameters are not known and would have to be determined first before finding a suitable packaging for this specific compound.

Liposomal applications have been widely discussed in medicine and pharmacology and some sophisticated solutions have been developed for specific active agents. Their use, however, is not very common. One reason are the relatively high production costs, another reason are possible adverse side effects. In particular, when parenterally applied, liposomes carry the risk of accumulating in the liver, the spleen and/or the bone marrow. Therefore, liposomal formulations are often viewed skeptically.

A nano-liposphere-based formulation method for increasing drug bioavailability was disclosed in WO2013/108254. Although this method offers some advancement over the state-of-the-art there are also some inherent drawbacks. High-pressure homogenizers are needed for the production of these solid lipid nanoparticles. However, high-pressure induced drug degradation has been described for some drugs or dietary supplements. Lipid crystallization, gelation phenomena and co-existence of several colloidal species occur. Further restrictive factors such as cytotoxic effects after phagocytosis, toxic effects of organic residues and a difficult industrial scale-up have limited their use. Moreover, their drug loading capacity is relatively small and they display a low viscosity. Further, the use of an amphiphilic solvent such as lower alkyl esters of lactic acid or N-methylpyrrolidone is required in WO2013/108254. N-methylpyrrolidone is listed as a substance of very high concern in respect of being potentially carcinogenic and toxic for reproduction, methyl lactate is usually hydrolyzed to lactate and methanol in an aqueous environment. Ethyl lactate is well tolerated. However due to relatively high production costs it is not a very attractive solvent, particularly not for dietary supplements.

The use of piperine as a chemosensitizing agent for a phospholipid-curcumin complex is revealed in EP2228062A1. An improvement of solubility is not addressed therein.

A phosphatidylcholine-based self-emulsification technique for solubilizing dietary supplements and pharmaceutically active agents has been disclosed in EP16001941.

Another solubilization technique is the formation of inclusion complexes of the substance to be solubilized with cyclodextrins such as a-, 13- or y-cyclodextrin or cyclodextrin derivatives such as 2-hydroxypropyl-(3-cyclodextrin, methyl-p-cyclodextrin or trimethyl-p-cyclodextrin. Typically, cyclodextrins are composed of 6 to 8 1,4-linked a-D-glucopyrano- sides forming macrocycles. Thus a water-soluble toroid (cone-shaped or bucket-shaped) structure is generated which is capable to host hydrophobic substances in its interior. The interior space is considerably less hydrophilic than the outside contacting the aqueous environment. Cyclodextrins are produced from starch by enzymatic treatment. They are loaded with the compound to be solubilized by dispersion. The compound to be solubilized can then be released by contacting these complexes with water, by pH or temperature changes, depending on the specific composition. However, the development of cyclodextrin is apparently not easy and relatively costly.

Confusing and even contradictory definitions can be found in the prior art. In order to avoid any ambiguity a solubilisate according to this invention is defined as follows: A solubilisate is the composition of the at least one substance to be solubilized and the solubilizing agents. Further addition of a solvent or diluent shall not be covered by this term. The solubilisate is produced first by a solubilization method, then a specific nutritional or pharmaceutical composition is produced with said solubilisate, and finally said nutritional or pharmaceutical composition is packaged into a suitable container for the respective product.

It is characterized by the substantially complete solubilization of the substance, thus being a nearly perfect solution in which the molecules behave completely as independent entities in a solution and substantially undergo the distribution and thermodynamic rules of Brownian motion. Thus the solubilisate is a clear solution containing the respective dietary supplement or pharmaceutically active agent in a high concentration. In general, the solubilisate is not meant for intake without dilution.

A solubilisate must be differentiated from a suspension (colloidal suspension). This term defines a heterogeneous mixture containing solid particles that sooner or later will undergo sedimentation. It is also different from an emulsion (a mixture of two liquids which usually are immiscible). For increasing the cell- or bioavailability and/or resorption of a substance the complete solubilization is highly preferably.

A solubilisate must also be differentiated from a concentrate. A concentrate is a compound, respectively a composition of compounds without a diluent. Upon release of a concentrate into a diluent the concentrate dissolves itself either completely in the diluent or forms a suspension or emulsion with the diluent. A concentrate does not need the interaction with solubilizing agents, as it is intrinsically solvable in water or an aqueous solution.

The term solubilisate must be differentiated from the finished solution, respectively the prepared beverage to be drunk. This finished solution is generated by diluting the solubilisate according to the invention in a diluent, preferably an aqueous solution, in order to produce a beverage, respectively any fluid dosage form ready for intake by the consumer, respectively the patient.

A diluent is a diluting agent (dilutant, thinner). It is not part of the solubilisate.

A great advantage of such a solubilisate consists in its small volume. Thus it can be easily portioned to patient- or consumer-friendly units, or relatively huge amounts of a solubilized substance can be shipped at low costs. In order to produce a finished solution the dilution of the solubilisate in an aqueous medium (e.g. tap water or still mineral water) can be easily carried out by medical staff, patients or consumers.

In a preferred embodiment according to the invention the Alpha Lipoic Acid solubilisate, Epigallocatechin 3-gallate solubilisate, and Curcuminoid solubilisates are provided in the overall range of 2 % to 15 % per weight, in a more preferred embodiment in the overall range of 2 % to 10 % per weight.

Phosphatidylcholines are a class of phospholipids linked to choline. They are a major component of cell membranes and are for example obtained from egg yolk, ox liver, marine animals or soybeans. In practice, it showed that the origin of phosphatidylcholines influences their biological and chemical effects considerably. According to the solubilisates of this invention at least one phosphatidylcholine (PC) can be selected from the group comprising 1-palmitoyl-2-oleoyl-sn-glycero-3-phosphocholine (POPC), natural (non-hydrogenated) soy bean PC, natural egg PC, dipalmitoyl phosphatidylcholine (DPPC), dimyristoyl phosphatidylcholine (DMPC) or 1,2-dioleyl-SN-glycero-3-phosphocholine (DOPC), 1-oleoyl-palmi-toyl phosphatidylcholine (OPPC), diasteroyl phosphatidylcholine (DSPC), monostearoylphosphatidylcholine (MSPC), diarachidoylphosphatidylcholine (DAPC), corn lecithin, cottonseed oil lecithin, rapeseed lecithin, phosphatidyleth- anolamine, phosphatidylserine, phosphatidylinositol, phosphatidylinositol polyphosphates, phosphatidylglycerol, phos- phatidic acid, phosphatidylinositolamine, diphosphatidylglycerol (cardiolipin), sphingomyelin, ceramide aminoethylphos- phonic acid, ceramide phosphorylglycerol, dicetylphosphoric acid, stearylamine, and mixtures thereof. Preferred phosphatidylcholines are non-hydrogenated phosphatidylcholines. Particularly preferred phosphatidylcholines are non-hydrogenated soybean PC, DMPC, POPC and DOPC. Most preferred is non-hydrogenated soybean PC. Lecithin is commonly used as a synonym for phosphatidylcholines. It is a mixture of phosphatidylcholine and other compounds. Phosphatidylcholines can be added in the overall range of 0.5% to 24.5% per weight.

Medium-chained triglycerides (MCT) refer to triglycerides whose fatty acids have an aliphatic tail of 6 - 12 carbon atoms. Fatty acids incorporated in MCT are called medium-chain fatty acids (MCFA). In triglycerides three fatty acid molecules are bound to a glycerol backbone. Per definition, in MCT at least two of these three fatty acids must be MCFAs. MCFA can be selected independently from one another from the group comprising caproic acid, enanthic acid, caprylic acid, pelargonic acid, capric acid, undecilyc acid, lauric acid, their unsaturated derivatives, and mixtures thereof. Preferred MCFA are caproic acid, caprylic acid, capric acid, and lauric acid. MCT oils or MCT fats are oils or fats containing predominantly said MCT. These terms refer to a respective mixture of different MCT that may contain a variety of MCFA. Accordingly any reasonable mixing ratio shall be covered by these terms. MCT fats are often extracted from specific plant fats, while MCT oils do not occur naturally. MCT oils and MCT fats are broadly marketed as a healthy dietary supplement, respectively as a surrogate for long-chain fats in nutrition. MCT are used in the overall range of 0.5% to 24.5% per weight.

For augmenting the shelf life of solubilisates containing at least one oxidation-prone dietary supplement or pharmaceutically active agent at least one further antioxidant can be added to the solubilisate. In preferred embodiments this at least one antioxidant is a food additive and/or a pharmaceutically acceptable excipient. Suitable antioxidants can be selected from the group comprising lactic acid, ascorbic acid, sodium ascorbate, calcium ascorbate, potassium ascorbate, fatty acid esters of ascorbic acid, ascorbyl palmitate, ascorbyl stearate, tocopherols, alpha-tocopherol, beta-tocopherol, gamma-tocopherol, delta-tocopherol, alpha-tocotrienol, beta-tocotrienol, gamma-tocotrienol, delta-tocotrienol, propyl gallate, octyl gallate, dodecyl gallate, ethyl gallate, guaiac resin, erythorbic acid, sodium erythorbate, erythorbin acid, sodium erythorbin, tert-butylhydroquinone, butylated hydroxyanisole, butylated hydroxytoluene, mono-, di-, trisodium phosphate, mono-, di-, tripotassium phosphate, anoxomer, ethoxyquin, potassium lactate, stannous chloride, sodium thiosulfate, 4-hexylresorcinol, glucose oxidase. Preferred are ascorbyl palmitate and alpha-tocopherol, beta-tocopherol, gamma-tocopherol, delta-tocopherol. Particularly preferred is a combination of ascorbyl palmitate and at least one of alpha-tocopherol, beta-tocopherol, gamma-tocopherol, delta-tocopherol. The term tocopherol refers to any of the aforementioned tocopherols or a mixture thereof. Most preferred pharmaceutically acceptable excipient of the present application is N-(2-Hydroxyethyl)hexadecanamide.

Thus the present application refers also to a method for solubilizing Alpha Lipoic Acid, Epigallocatechin 3-gallate, and Curcumin, comprising the following steps: Providing at least one pharmaceutically active agent or dietary supplement in the overall range of 0.5 % to 25 % per weight at room temperature and a pressure of 0.2 bar to 1 bar;

Adding at least one oil extract in the overall range of 75 % to 99 % per weight, and at least one of phosphatidylcholines in the overall range of 0.5 % to 24.5 % per weight, lysophosphatidylcholines in the overall range of 0.5 % to 10 % per weight, medium-chained triglycerides in the overall range of 0.5 % to 24.5 % per weight, C₂ to C₄ alcohols in the overall range of 0.5 % to 5 % per weight, glyceryl stearate in the overall range of 0.5 % to 3 % per weight, saturated or unsaturated C₁₄ to C₂₀ fatty acids in the overall range of 0.5 % to 3 % per weight, and antioxidants, most preferred N-(2-Hydroxyethyl)hexadecanamide, in the overall range of 0.01 % to 3 % per weight, wherein the relative weight percentages of the at least one pharmaceutically active agent or dietary supplement and the at least one oil extract add up to 100%;

Cautiously heating the resulting mixture by continuously increasing the temperature with a continuous temperature increment of 0.5°C/min - 3°C/min over a period of 20 - 60 minutes;

Stopping the temperature increase in a temperature range of 30°C to 125°C as soon as a clear solution is reached; and

Letting the resulting solubilisate cool down to room temperature.

Accordingly, the invention relates to, inter alia, the following embodiments:
1. A composition comprising the Alpha Lipoic Acid solubilisate, Epigallocatechin 3-gallate solubilisate, and Curcumin solubilisate for use of embodiment 1, for use in the treatment and prevention of arthritis.
2. A pharmaceutical product comprising the Alpha Lipoic Acid solubilisate, Epigallocatechin 3-gallate solubilisate, and Curcumin solubilisate or the composition for use of embodiment 2, and a pharmaceutically acceptable carrier, excipient (most preferred N-(2-Hydroxyethyl)hexadecanamide) and/or diluents for use in the treatment and prevention of arthritis.

In addition, the invention relates to, inter alia, the following embodiments:
1. A composition comprising the Alpha Lipoic Acid solubilisate, Epigallocatechin 3-gallate solubilisate, and Curcumin solubilisate for use in the treatment and prevention of viral infections, like SARS-CoV-2 virus infection.
2. A pharmaceutical product comprising the Alpha Lipoic Acid solubilisate, Epigallocatechin 3-gallate solubilisate, and Curcumin solubilisate, and a pharmaceutically acceptable carrier, excipient (most preferred N-(2-Hydroxyethyl)hexadecanamide) and/or diluents for use for use in the treatment and prevention of viral infections, like SARS-CoV-2 virus infection.

Furthermore, the invention relates to the inhalative use of the Alpha Lipoic Acid solubilisate, Epigallocatechin 3-gallate solubilisate, and Curcumin or Terathydrocurcumin solubilisates in combination as therapeutic medicament for viral infections of the respiratory tract as exemplified for SARS-CoV-2.

Pulmonary diseases affect the lower airways of the respiratory system, in particular the lungs. This term encompasses pathological conditions that impair the gas exchange in the lungs or bronchi in mammals. In general, they are differentiated into obstructive and restrictive pulmonary diseases. Obstructive pulmonary diseases are characterized by airway obstruction. This limits the amount of air that is able to enter the alveoli because of constriction of the bronchial tree, due to inflammation. Restrictive pulmonary diseases are characterized by a loss of lung compliance, causing incomplete lung expansion and increased lung stiffness.

They can be also categorized as airway diseases, lung tissue diseases, lung circulation diseases, lung infectious diseases and lung proliferative diseases. Airway diseases affect the tubes that carry oxygen and other gases into and out of the lungs. They usually cause a narrowing or blockage of the airways. Typical airway diseases include asthma, chronic obstructive pulmonary disease and bronchiectasis. Lung tissue diseases affect the structure of the lung tissue. Scarring or inflammation of the tissue makes the lungs unable to expand fully. This complicates the gas exchange. As a result, these patients can't breathe deeply. Pulmonary fibrosis and sarcoidosis are typical examples thereof. Lung circulation diseases affect the blood vessels of the lung. They are caused by clotting, scarring, or inflammation of the blood vessels. They affect the gas exchange and possibly also heart function. A typical example is pulmonary hypertension. Lung infectious diseases refer to disorders caused by an infection of the lower airways, e.g. pneumonia. Most of the airway diseases are caused by an underlying inflammation or at least include an inflammatory component. Lung tissue diseases have often also an inflammatory component, unless they are caused by direct physical impairment of the respiratory tract. Lung circulation diseases such as pulmonary hypertension have in general an inflammatory component at the affected vessel section. Infectious and proliferative diseases of the lungs may also have an inflammatory component, often secondary to the infection or the underlying malignancy.

Thus, these inflammatory pulmonary diseases have in common that they could be pharmacologically treated by anti-inflammatory drugs. However, therapeutic efficacy is often hampered by insufficient availability, respectively efficacy of the drug at the site of inflammation in the lung. Systemic administration, e.g. orally or parenterally, yields often no or only an insufficient therapeutic success.

An alternative administration route is inhalation. Metered-dose inhalers (MDI) are widely in use, e.g. in the treatment of asthma. They usually have a container, respectively canister for the pharmaceutical formulation, a metering valve, for metering the dispensed quantity and a mouthpiece for inhaling. The pharmaceutical formulation consists of the drug, a liquefied gas propellant such as hydrofluoroalkanes and optionally pharmaceutically acceptable excipients.

A specific group of MDIs are dry powder inhalers (DPI). They deliver the drug to the lungs in the form of a dry powder. Most DPIs rely on the force of patient inhalation to entrain powder from the device and subsequently break-up the powder into particles that are small enough to reach the lungs. For this reason, insufficient patient inhalation flow rates may lead to reduced dose delivery and incomplete disaggregation of the powder, leading to unsatisfactory device performance. Thus, most DPIs need a minimum inspiratory effort for proper use. Therefore, their use is limited to older children and adults.

While disorders affecting the bronchi or upper parts of the lower airways can be addressed this way, e.g. by asthma sprays, disorders affecting the alveoli where the gas exchange takes place can be only insufficiently treated because of ineffective inhalatory administration, e.g. COPD. The administered drug particles are not able to reach the bottom of the lungs by way of inhalation, at least not in a therapeutically effective amount.

Nebulizers use to administer the active principle in the form of a mist inhaled into the lungs. Physically, this mist is an aerosol. It is generated in the nebulizer by breaking up solutions and suspensions into small aerosol droplets (preferred) or solid particles that can be directly inhaled from the mouthpiece of the device. In conventional nebulizers the aerosol can be generated by mechanical force, e.g. spring force in soft mist nebulizers, or electrical force. In jet nebulizers a compressor brings oxygen or compressed air to flow at high velocity through the aqueous solution with the active principle, this way generating an aerosol. A variant are pressurized metered-dose inhalers (pMDIs). Ultrasonic wave nebulizers use an electronic oscillator that at high frequency causes vibration of a piezoelectric element for generating ultrasonic waves in the liquid reservoir with the active principle.

The most promising technology are vibrating mesh nebulizers. They use a mesh, respectively a polymer membrane having a very large number of usually laser-drilled holes. This membrane is placed between the liquid reservoir and the aerosol chamber. A piezoelectric element placed on the membrane induces high frequency vibrations of the membrane, leading to droplet formation in the aqueous solution and pressuring these droplets through the holes of the membrane into the aerosol chamber. With this technique very small droplet sizes can be generated. Moreover, a significantly shorter inhalation time for the patient can thus be achieved, a feature which drastically increases patient compliance. Only these mesh nebulizers are regarded to be able to generate liquid droplets with the active principle in the desired size range and bring them in a therapeutically effective amount into the patient's alveoli in a reasonable time.

However, not all agents that might be effective in the pharmaceutic treatment of inflammatory pulmonary diseases are suitable for the mesh nebulization technology. For example, some agents are nearly insoluble in water, or their intrinsic physicochemical molecule properties do not allow for the generation of an aerosol in the desired particle size range. Therefore, many nebulized anti-inflammatory agents met only mixed success in the treatment of inflammatory pulmonary diseases.

Thus, there is a medical need to find pharmaceutical agents that show a high efficacy in the treatment of inflammatory pulmonary diseases and at the same time allow for the generation of an aerosol in the desired liquid droplet or solid particle size range in order to be able to reach the alveoli of a patient in need thereof.

In the scope of the present application the terms "alveoli" and "alveolar" refer to the tissue structures at the bottom of the lung airways. Alveoli are hollow cup-shaped cavities found in the lung parenchyma where gas exchange takes place. Further, they are located sparsely on the respiratory bronchioles, line the walls of the alveolar ducts, and are more numerous in the blind-ended alveolar sacs. The alveolar membrane is the gas exchange surface, surrounded by a network of capillaries. Across the membrane oxygen is diffused into the capillaries and carbon dioxide released from the capillaries into the alveoli to be breathed out. Alveoli consist of an epithelial layer of simple squamous epithelium and an extracellular matrix surrounded by capillaries. The epithelial lining is part of the alveolar membrane, also known as the respiratory membrane.

Type I and type II pneumocytes are found in the alveolar wall. Alveolar macrophages are immune cells that move about in the alveolar lumen and in the connective tissue between them. Type I cells are squamous epithelial cells, thin and flat and form the structure of the alveoli. Type II cells (goblet cells) release pulmonary surfactant to lower surface tension.

A typical pair of human lungs contain about 300 million alveoli, producing 70 m² of surface area. Each alveolus is wrapped in a fine mesh of capillaries covering about 70% of its area. The diameter of a typical healthy alveolus is between 200 and 500 µm.

Inflammatory pulmonary diseases (preferred in the embodiments of the present invention) can be classified as follows (according to ICD-10 Chapter X: Diseases of the respiratory system (J00-J99), Version 2016, as of January 10^{th}, 2020):
a) Inflammation of the lower airways due to a bacterial, viral, fungal or parasitic infection

These diseases comprise, without being limiting, influenza due to identified avian or human influenza virus; influenza with pneumonia, adenoviral pneumonia; pneumonia due to Corona virus infection; pneumonia due to *Streptococcus* pneumoniae; pneumonia due to *Haemophilus* influenzae; pneumonia due to *Klebsiella* pneumoniae; pneumonia due to *Pseudomonas;* pneumonia due to *Staphylococcus;* pneumonia due to *Streptococcus,* group B; pneumonia due to other streptococci; pneumonia due to *Escherichia coli;* pneumonia due to other aerobic Gram-negative bacteria; pneumonia due to *Mycoplasma pneumoniae;* other bacterial pneumonia; pneumonia in mycoses; pneumonia in parasitic diseases; acute bronchitis; acute bronchiolitis.

For administration by inhalation the particle diameter of the solubilized medicament preparation is preferably between 2 to 10 µm, more preferably between 3 to 7 µm. The solubilized medicament preparation is particularly suitable for administration using an inhaler, for example commercially available mesh nebulizers comprising, without being limiting, PARI eFlow rapid, PARI LC STAR, PARI Velox and PARI Velox Junior (PARI GmbH, Starnberg, Germany), Philips Respironics I-neb and Philips InnoSpire Go (Koninklijke Philips N.V., Eindhoven, Netherlands), VENTA-NEB-ir, OPTI-NEB, M-neb dose+ mesh nebulizer MN-300/8 or 300/9, M-Neb Flow+ and M-neb mesh nebulizer MN-300/X (NEBU-TEC, Eisenfeld, Germany), Hcmed Deepro HCM-86C and HCM860 (HCmed Innovations Co., Ltd, Taipei, Taiwan), OMRON MicroAir U22 and U100 (OMRON, Kyoto, Japan), Aerogen Solo, Aerogen Ultra and AerogenPRO (Aerogen, Galway, Ireland), KTMED NePlus NESM1 (KTMED Inc., Seoul, South Korea), Vectura Bayer Breelib (Bayer AG, Leverkusen, Germany), MPV Truma and MicroDrop Smarty (MPV MEDICAL GmbH, Kirchheim, Germany), MOBI MESH (APEX Medical, New Taipei City, Taiwan), B.Well WN-114, TH-134, and TH-135 (B.Well Swiss AG, Widnau, Switzerland), Babybelle Asia BBU01 (Babybelle Asia Ltd., Hongkong), CA-MI Kiwi and others (CA-MI sri, Langhirano, Italy), Diagnosis PRO MESH (Diagnosis S.A., Biatystok, Poland), DIGI 02 (DigiO2 International Co., Ltd., New Taipei City, Taiwan), feellife AIR PLUS, AEROCENTRE+, AIR 360+, AIR GARDEN, AIRICU, AIR MASK, AIRGEL BOY, AIR ANGEL, AIRGEL GIRL and AIR PRO 4 (Feellife Health Inc., Shenzhen, China), Hannox MA-02 (Hannox International Corp., Taipei, Taiwan), Health and Life HL100 and HL100A (HEALTH & LIFE Co., Ltd., New Taipei City, Taiwan), Honsun NB-810B (Honsun Co., Ltd., Nantong, China), K-jump KN-9100 (K-jump Health Co., Ltd., New Taipei City, Taiwan), microlife NEB-800 (Microlife AG, Widnau, Switzerland), OK Biotech Docspray (OK Biotech Co., Ltd., Hsinchu City, Taiwan), Prodigy Mini-Mist (Prodigy Diabetes Care, LLC, Charlotte, USA), Quatek NM211, NE203, NE320 and NE403 (Big Eagle Holding Ltd., Taipei, Taiwan), Simzo NBM-1 and NBM-2 (Simzo Electronic Technology Ltd., Dongguan, China), Mexus BBU01 and BBU02 (Tai Yu International Manufactory Ltd., Dongguan, China), TaiDoc TD-7001 (TaiDoc Technology Co., New Taipei City, Taiwan), Vibralung and HIFLO Miniheart Circulaire II (Westmed Medical Group, Purchase, USA), KEJIAN (Xuzhou Kejian Hi-Tech Co., Ltd., Xuzhou, China), YM-252, P&S-T45 and P&S-360 (TEKCELEO, Valbonne, France), Maxwell YS-31 (Maxwell India, Jaipur, India), Kernmed JLN-MB001 (Kernmed, Durmersheim, Germany), Yuwell M102 (Yuwell, Nanjing, China), Scian NB-812B (Scian, Shanghai, China).

Preferred are mesh nebulizers with a piezoelectric activation of the nebulization process, respectively vibrating mesh nebulizers.

Mesh nebulizers can be classified into two groups according to patient interaction: Continuous mode devices and trigger-activated devices. In continuous mode mesh nebulizers the nebulized aerosol is continuously released into the mouth piece and the patient has to inhale the provided aerosol. In trigger-activated devices a defined amount of aerosol is released only upon an active and deep inspiratory breath.

The most preferred mesh nebulizers are models such as feellife 360+, PARI eFlow^{®}rapid, Philips Respironics I-neb, M-neb^{®} MN-300/9, Vectura Bayer Breelib^{™}.

The solubilized product can be mixed in water or any other suitable liquid for inhalation administration.

A "pharmaceutically acceptable carrier, excipient and/or diluent", as used herein, refers to an ingredient in the pharmaceutical product, other than the active ingredient(s), which is nontoxic to recipients at the dosages and concentrations employed.

As used herein the term "combination administration" of a compound, therapeutic agent or known drug with a medication of the present invention means administration of the drug and the one or more compounds at such time that both the known drug and the medication will have a therapeutic effect. In some cases, this therapeutic effect will be synergistic. Such concomitant administration can involve concurrent (i.e., at the same time), prior, or subsequent administration of the drug with respect to the administration of a medication of the present invention. A person of ordinary skill in the art would have no difficulty determining the appropriate timing, sequence and dosages of administration for particular drugs and medications of the present invention.

Accordingly, the invention is at least in part based on the surprising finding, that the means and methods of the invention can be used in the prevention and/or treatment of arthritis.

### Pharmaceutical compositions

Still another aspect of the present invention relates to the use of a combination of three active compounds as active ingredients, together with at least one pharmaceutically acceptable carrier, excipient and/or diluents for the manufacture of a pharmaceutical composition for the treatment and/or prophylaxis of an autoimmune disease, a fibrotic disease, an inflammatory disease, such as arthritis, and viral disease such as Corona-virus infections.

Such pharmaceutical compositions comprise a combination of three active compounds as active ingredients, together with at least one pharmaceutically acceptable carrier, excipient, binders, disintegrates, glidants, diluents, lubricants, colouring agents, sweetening agents, flavouring agents, preservatives or the like. The pharmaceutical compositions of the present invention can be prepared in a conventional solid or liquid carrier or diluents and a conventional pharmaceutically-made adjuvant at suitable dosage level in a known way.

Preferably the combination of three active compounds is suitable for oral administration, and inhalative administration.

Administration forms include, for example, pills, tablets, film tablets, coated tablets, capsules, oral drink formulations, micro- and nano-formulations, powders and deposits. Furthermore, the present invention also includes pharmaceutical preparations for oral application, which preparations in addition to typical vehicles and/or diluents contain a combination of three active compounds according to the present invention.

The combination of three active compounds of the invention forms pharmaceutically acceptable salts with organic and inorganic acids. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphersulfonic acid, china acid, mandelic acid, o-methylmandelic acid, hydrogen-benzenesulfonic acid, picric acid, adipic acid, D-o-tolyltartaric acid, tartronic acid, □-toluic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

The pharmaceutical compositions according to the present invention will typically be administered together with suitable carrier materials selected with respect to the intended form of administration, i.e., for oral administration in the form of solubilisates, tablets, capsules (either solid filled, semi-solid filled or liquid filled), powders for constitution, aerosol preparations consistent with conventional pharmaceutical practices. Other suitable formulations are gels, elixirs, dispersible granules, syrups, suspensions, creams, lotions, solutions, emulsions, dispersions, and the like.

Additionally, the compositions of the present invention may be formulated in sustained release solubilisate form to provide the rate-controlled release of any one or more of the components or active ingredients to optimize the therapeutic effects. Liquid form preparations include solubilisates, solutions, suspensions and emulsions.

The manufactured medicaments of the invention comprise:
a) A daily dose of 165 milligrams of Alpha Lipoic Acid solubilisate, 300 milligrams of EGCG solubilisate, 105 milligrams of Curcumin solubilisate orally administered as a drink.
b) A daily dose of 165 milligrams of Alpha Lipoic Acid solubilisate, 105 milligrams of Curcumin solubilisate, and 300 milligrams of EGCG solubilisate split in three inhalations.
c) A daily dose of 165 milligrams of Alpha Lipoic Acid solubilisate, 105 milligrams of Tetrahydrocurcumin solubilisate, and 300 milligrams of EGCG solubilisate split in three inhalations.

### Method of treatment

Another aspect of the present invention relates to a method of prophylaxis and/or treatment of arthritis comprising administering to a patient in need thereof a pharmaceutical composition comprising a drug combination according to the present invention.

Accordingly, the terms "prophylaxis" or "treatment" includes the administration of the drug combination of the present invention to prevent, inhibit, or arrest the symptoms of arthrits. In some instances, treatment with the drug combination of the present invention will be done in combination with other protective compounds to prevent, inhibit, or arrest the symptoms of arthritis.

The term "active agent" or "therapeutic agent" as used herein refers to an agent that can prevent, inhibit, or arrest the symptoms and/or progression of arthritis.

The term "therapeutic effect" as used herein, refers to the effective provision of protection effects to prevent, inhibit, or arrest the symptoms and/or progression of arthritis.

The term "a therapeutically effective amount" as used herein means a sufficient amount of one or more of the drug candidates of the invention to produce a therapeutic effect, as defined above, in a subject or patient in need of treatment.

The terms "subject" or "patient" are used herein mean any mammal, including but not limited to human beings, including a human patient or subject to which the compositions of the invention can be administered.

The drug combination of the present invention can be used for the prophylaxis and/or treatment of arthritis in combination administration with another therapeutic compound. As used herein the term "combination administration" of a compound, therapeutic agent or known drug with the drug combination of the present invention means administration of the drug and the one or more compounds at such time that both the known drug and drug combination will have a therapeutic effect. In some cases, this therapeutic effect will be synergistic. Such concomitant administration can involve concurrent (i.e., at the same time), prior, or subsequent administration of the drug with respect to the administration of the drug combination of the present invention. A person of ordinary skill in the art would have no difficulty determining the appropriate timing, sequence and dosages of administration for particular drugs and drug combination of the present invention.

### Drugs of the combination

The molecules having the IUPAC names: (*R*)-5-(1,2-Dithiolan-3-yl)pentanoic acid, (or Alpha Lipoic Acid), Curcumin and Epigallocatechin 3-gallate.

Furthermore, the present invention relates to the use of the above-mentioned drug solubilisates as pharmaceutically active agents in medicine, i.e., as medicament.

"a," "an," and "the" are used herein to refer to one or to more than one (i.e., to at least one, or to one or more) of the grammatical object of the article.

"or" should be understood to mean either one, both, or any combination thereof of the alternatives.

"and/or" should be understood to mean either one, or both of the alternatives. Throughout this specification, unless the context requires otherwise, the words "comprise", "comprises" and "comprising" will be understood to imply the inclusion of a stated step or element or group of steps or elements but not the exclusion of any other step or element or group of steps or elements.

Reference throughout this specification to "one embodiment," "an embodiment," "a particular embodiment," "a related embodiment," "a certain embodiment," "an additional embodiment," "a specific embodiment" or "a further embodiment" or combinations thereof means that a particular feature, structure or characteristic described in connection with the embodiment is included in at least one embodiment of the present invention. Thus, the appearances of the foregoing phrases in various places throughout this specification are not necessarily all referring to the same embodiment. Furthermore, the particular features, structures, or characteristics may be combined in any suitable manner in one or more embodiments. It is also understood that the positive recitation of a feature in one embodiment, serves as a basis for excluding the feature in a particular embodiment.

The invention will be further described in the following examples, which do not limit the scope of the invention described in the claims.

### EXAMPLES

The drug compounds as listed above were applied to patients in need after suffering from arthrosis.

### EXAMPLE 1:

We manufactured an solubilized version of Epigallocatechin 3-gallate (EGCG) which can reach the cells in the joints and regenerate the disturbed mitochondria in order to regenerate the ATP production in diseased cells. The following indications refer to the weight percent of the mixture. A solubilisate of ca. 20 ml was generated. EGCG (Sabinsa, Langen, Germany) was provided, and then the MCT oil extract and the additional agents were admixed one by one under stirring for 5 min at room temperature (20°C) and atmospheric pressure. The phosphatidylcholine used herein was purchased from Lipoid GmbH, Ludwigshafen, Germany.

Then the composition was cautiously heated under continued stirring, with an approximate temperature increment of 2°C /min. After ca. 35 min (ca. 90°C) the composition started to become a clear solution. This solubilization process lasted for additional 7 min. Thus a EGCG solubilisate according to the invention was obtained after ca. 42 min at ca. 105°C. Then the heating and the stirring was stopped and the resulting solubilisate was allowed to cool down to room temperature. The color of the solubilisate was dark brownish. The solubilisate stayed clear and stable over min. 16 months. Upon being diluted into an aqueous finished solution (2 ml solubilisate added to 200 ml water) under stirring the finished solution became quickly clear and had a pale brownish appearance.

### EXAMPLE 2:

We manufactured an solubilized version of Alpha Lipoic Acid (ALA) which can reach the cells in the joints and regenerate the disturbed mitochondria in order to regenerate the ATP production in diseased cells. The following indications refer to the weight percent of the mixture. A solubilisate of ca. 20 ml was generated. ALA (Sabinsa, Langen, Germany) was provided, and then the MCT oil extract and N-(2-Hydroxyethyl)hexadecanamide was admixed one by one under stirring for 5 min at room temperature (20°C) and atmospheric pressure. The phosphatidylcholine used herein was purchased from Lipoid GmbH, Ludwigshafen, Germany. As an additional excipient, Polyoxyethylen-sorbitan-monooleat can be added.

Then the composition was cautiously heated under continued stirring, with an approximate temperature increment of 2°C /min. After ca. 35 min (ca. 90°C) the composition started to become a clear solution. This solubilization process lasted for additional 5 min. Thus a ALA solubilisate according to the invention was obtained after ca. 40 min at 105°C. Then the heating and the stirring was stopped and the resulting solubilisate was allowed to cool down to room temperature. The color of the solubilisate was silver. The solubilisate stayed clear and stable over min. 14 months. Upon being diluted into an aqueous finished solution (2 ml solubilisate added to 200 ml water) under stirring the finished solution became quickly clear and had a pale silver appearance.

### EXAMPLE 3:

We manufactured an solubilized version of Curcumin which can reach the cells in the joints and regenerate the disturbed mitochondria in order to regenerate the ATP production in diseased cells. The following indications refer to the weight percent of the mixture. A solubilisate of ca. 20 ml was generated. Curcumin (Sabinsa, Langen, Germany) was provided, and then the MCT oil extract and the additional agents were admixed one by one under stirring for 5 min at room temperature (20°C) and atmospheric pressure. The phosphatidylcholine used herein was purchased from Lipoid GmbH, Ludwigshafen, Germany.

Then the composition was cautiously heated under continued stirring, with an approximate temperature increment of 2°C /min. After 30 min (ca. 90°C) the composition started to become a clear solution. This solubilization process lasted for additional 5 min. Thus a Curcumin solubilisate according to the invention was obtained after 35 min at 105°C. Then the heating and the stirring was stopped and the resulting solubilisate was allowed to cool down to room temperature. The color of the solubilisate was yellow. The solubilisate stayed clear and stable over min. 12 months. Upon being diluted into an aqueous finished solution (2 ml solubilisate added to 200 ml water) under stirring the finished solution became quickly clear and had a strong yellow appearance.

### EXAMPLE 4:

We manufactured an solubilized version of Tetrahydrocurcumin which can reach the cells in the joints and regenerate the disturbed mitochondria in order to regenerate the ATP production in diseased cells. The following indications refer to the weight percent of the mixture. A solubilisate of ca. 20 ml was generated. Tetrahydrocurcumin (Sabinsa, Langen, Germany) was provided, and then the MCT oil extract and N-(2-Hydroxyethyl)hexadecanamide were admixed one by one under stirring for 5 min at room temperature (20°C) and atmospheric pressure. The phosphatidylcholine used herein was purchased from Lipoid GmbH, Ludwigshafen, Germany.

Then the composition was cautiously heated under continued stirring, with an approximate temperature increment of 2°C /min. After 30 min (ca. 90°C) the composition started to become a clear solution. This solubilization process lasted for additional 5 min. Thus a Tetrahydrocurcumin solubilisate according to the invention was obtained after 35 min at 105°C. Then the heating and the stirring was stopped and the resulting solubilisate was allowed to cool down to room temperature. The color of the solubilisate was white. The solubilisate stayed clear and stable over min. 12 months. Upon being diluted into an aqueous finished solution (2 ml solubilisate added to 200 ml water) under stirring the finished solution became quickly clear and had a white appearance.

### EXAMPLE 5:

### SARS-CoV-2 inhibition assay

The virus strain SARS-CoV-2 was isolated from a male patient and amplified in Vero B4. Viral titers were determined by an endpoint titration assay.

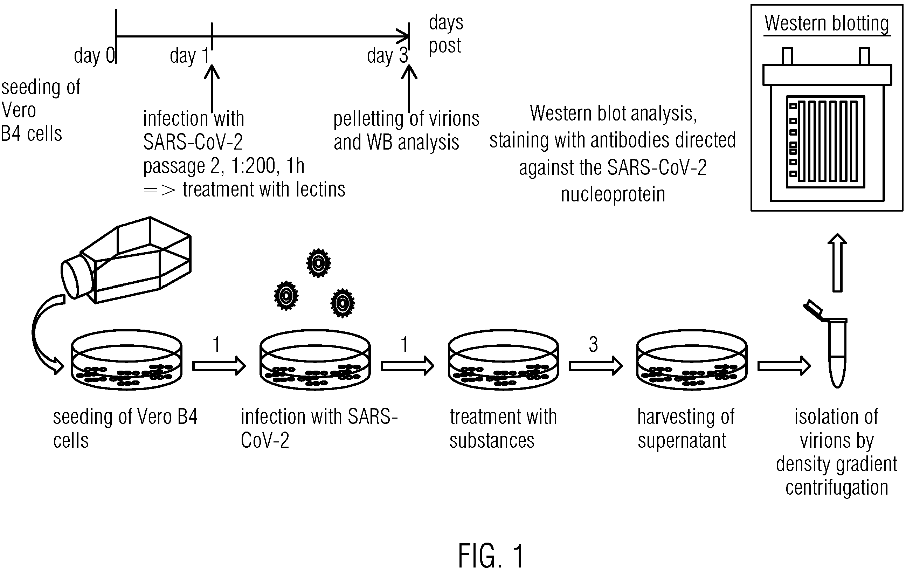

Fig. 1 shows a flowchart of a Virus Inhibition Assay. As shown in Fig. 1, for Western Blot analysis, Vero B4 cells were infected with SARS-CoV-2 (multiplicity of infection = 0.02) for 1 h, washed and further treated with solubilized drug sample interventions. 72 hours post infection, virus-containing cell culture supernatants were harvested and released virions were purified through 20% (w/v) sucrose cushion.

Vero B4 cells were maintained in DMEM containing 10% FCS, 2 mM L-glutamine, 100 U/mL penicillin, and 100 µg/mL streptomycin. Confluent monolayers of Vero B4 cells were infected in FCS free DMEM with SARS-CoV-2. 72 hours post infection, cells were lysed in radio immunoprecipitation assay buffer (150 mM NaCl, 50 mM Tris-HCI pH 8.0, 1% NP-40, 0.5% Na-deoxycholate, 0.1% SDS, 10 mM EDTA, 5 mM NEM, and 1 mM PMSF and further used for Western Blot analysis.

### SDS-PAGE and Western blotting

Protein samples were separated by SDS-PAGE, transferred onto nitrocellulose membranes, blocked with 3% BSA and incubated with the appropriate primary antibody. Viral proteins were detected by antibodies derived from convalescent SARS-CoV-2 patient sera. The anti-human secondary antibody coupled to horseradish peroxidase was obtained from Dianova, Hamburg, Germany.

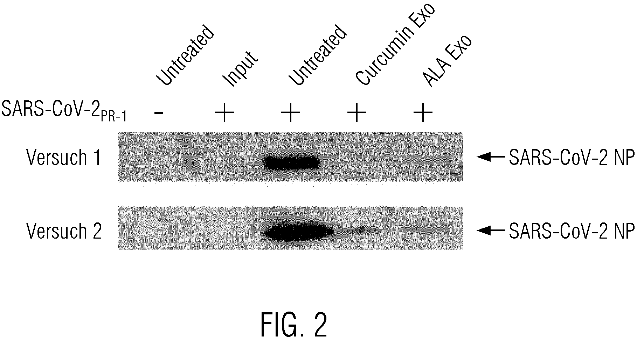

As shown in Fig. 2, in two different experiments (Versuch 1 and Versuch 2), the solubilized products of the Example 2 (solubilized Alpha Lipoic Acid), and of the Example 3 (solubilized Curcumin) strongly inhibited SARS-CoV-2 nucleoprotein production at physiological conditions of the solubilized products, and stopped the viral spread.

### EXAMPLE 6:

We performed a clinical observational study, including 26 patients with primary arthritis of the knee or coxarthrosis administering to each patient solubilized Alpha Lipoic Acid, Curcumin and EGCG once a day over a period of 8 weeks. The oldest subject was 90 years old, the youngest 29. The average age was 65 years. Before the start of the study, the pain intensity on the visual analog scale and the pain-free walking distance were recorded. The statistical classification was carried out for the pain intensity using the Wilcoxon test in Spss. The change in walking distance was assessed in Spss using t-test analysis.

After completing the study, a reduction in pain perception was documented in our patients cohort.

For the test parameter pain intensity, a strong decreas of Wilcoxon z-value by -3.836 was observed, the resulting effect size being 0.7523.

In the present cohort, the effect size is 0.7523> 0.5, which is a strong effect in terms of the difference in pain intensity before and after the end of the observation period.

(Effect limits between 0.1-0.3 are considered weak; 0.3-0.5 are considered average; greater than 0.5 are considered strong effects, rated according to Cohen).

The mean, pain-free walking distance before the start of therapy was 1268.27 m, after the study period increased significantly to 2036.54 m.

The results of this study show a dramatic and statistically significant improvement of the primary arthritis of the knee or coxarthrosis after oral treatment with the solubilized combination of the invention.

## Claims

1. Solubilized Alpha Lipoic Acid, solubilized Epigallocatechin 3-gallate, and solubilized Curcumin in combination as a therapeutic medicament for use in the treatment of arthritis.

2. A pharmaceutical product comprising solubilized Alpha Lipoic Acid, solubilized Epigallocatechin 3-gallate, and solubilized Curcumin in combination and a pharmaceutically acceptable carrier, N-(2-Hydroxyethyl)hexadecanamide as excipient, and/or diluents for use in the treatment of arthritis.

3. Solubilized Alpha Lipoic Acid, solubilized Epigallocatechin 3-gallate, and solubilized Curcumin in combination as a therapeutic medicament for use in the treatment of SARS-CoV-2 infection.

4. A pharmaceutical product comprising solubilized Alpha Lipoic Acid, solubilized Epigallocatechin 3-gallate, and solubilized Curcumin in combination and a pharmaceutically acceptable carrier, N-(2-Hydroxyethyl)hexadecanamide as excipient, and/or diluents for use in the treatment of SARS-CoV-2 infection.

5. Solubilized Alpha Lipoic Acid, solubilized Epigallocatechin 3-gallate, and solubilized Curcumin in combination or the pharmaceutical product for use of claim 2, wherein the treatment at least one disease or disorder selected from the group of an autoimmune disease, a fibrotic disease, an inflammatory disease, a neurodegenerative disease, with arthritic involvement.

6. Solubilized Alpha Lipoic Acid, solubilized Epigallocatechin 3-gallate, and solubilized Curcumin in combination or the pharmaceutical product for use of claim 2, wherein the arthritis is rheumatoid arthritis, arthrosis, osteoarthritis, gout, systemic lupus erythematodus.

7. Solubilized Alpha Lipoic Acid, solubilized Epigallocatechin 3-gallate, and solubilized Curcumin in combination for use in treatment of at least one symptom of arthritis selected from the group of pain, pain-free walking, and pain-free physical exercise.

8. Solubilized Alpha Lipoic Acid, solubilized Epigallocatechin 3-gallate, and solubilized Curcumin in combination for use of any of the previous claims, the composition for use of any of the previous claims or the pharmaceutical product for use of any of the previous claims, for use in oral treatment.

9. Solubilized Alpha Lipoic Acid, solubilized Epigallocatechin 3-gallate, and solubilized Curcumin in combination for use of any of the previous claims, the composition for use of any of the previous claims or the pharmaceutical product for use of any of the previous claims, for use in inhalation treatment.
